(19) Europäisches Patentamt
European Patent Office
Office européen des brevets

(11) EP 0 778 839 B1

(12) # EUROPÄISCHE PATENTSCHRIFT

(45) Veröffentlichungstag und Bekanntmachung des Hinweises auf die Patenterteilung:
**23.12.1998 Patentblatt 1998/52**

(21) Anmeldenummer: **95931211.7**

(22) Anmeldetag: **26.08.1995**

(51) Int Cl.6: **C07F 9/58**, A61K 31/675, C07F 9/6509

(86) Internationale Anmeldenummer:
**PCT/EP95/03382**

(87) Internationale Veröffentlichungsnummer:
**WO 96/06849 (07.03.1996 Gazette 1996/11)**

(54) **NEUE PHOSPHANOXIDE, VERFAHREN ZU IHRER HERSTELLUNG SOWIE DIESE VERBINDUNGEN ENTHALTENDE ARZNEIMITTEL**

NOVEL PHOSPHANE OXIDES, METHOD OF PREPARING THEM AND DRUGS CONTAINING THESE COMPOUNDS

NOUVEAUX OXYDES DE PHOSPHANE, LEUR PROCEDE DE FABRICATION ET MEDICAMENTS CONTENANT CES COMPOSES

(84) Benannte Vertragsstaaten:
**AT BE CH DE DK ES FR GB GR IE IT LI LU NL PT SE**

(30) Priorität: **30.08.1994 DE 4430755**

(43) Veröffentlichungstag der Anmeldung:
**18.06.1997 Patentblatt 1997/25**

(73) Patentinhaber: **BOEHRINGER MANNHEIM GMBH
68298 Mannheim (DE)**

(72) Erfinder:
- **VON DER SAAL, Wolfgang
D-69469 Weinheim (DE)**
- **LEINERT, Herbert
D-64646 Heppenheim (DE)**
- **STEGMEIER, Karlheinz
D-64646 Heppenheim (DE)**

(56) Entgegenhaltungen:
**EP-A- 0 555 824** **WO-A-94/20467**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 778 839 B1

Printed by Jouve, 75001 PARIS (FR)

## Beschreibung

Die Erfindung betrifft neue Phosphanoxide der allgemeinen Formel I

(I) 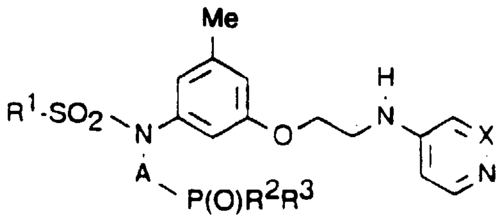

in der

| | |
|---|---|
| $R^1$ | eine Aryl- oder Heteroarylgruppe bedeutet, wobei die Aryl- oder Heteroarylreste ein- oder mehrfach durch Nitro, Halogen, Nitril, Hydroxy, Carboxy, Alkoxycarbonyl, Phenylalkoxycarbonyl, Phenyl, Alkyl, Trifluormethyl, Alkoxy, Alkenyloxy, Alkinyloxy, Aralkyloxy, Alkylthio, Alkylsulfinyl, Alkylsulfonyl, Amino, Alkylamino, Dialkylamino, Aralkylamino, Diaralkyl-amino, Alkylsulfonylamino, Alkylcarbonylamino, Formylamino, Carbamoyl, Thiocarbamoyl, Alkylaminocarbonyl, Dialkylaminocarbonyl oder Alkoxycarbonylalkyloxy substituiert sein können, |
| $R^2$ und $R^3$ | gleich oder verschieden sind und geradkettige oder verzweigte Alkylgruppen bedeuten, |
| A | einen geradkettigen oder verzweigten Alkylenrest bedeutet, |
| X | die -CH-Gruppe oder ein Stickstoffatom bedeutet, |

sowie Hydrate, Solvate und physiologisch verträgliche Salze davon. Gegenstand der Erfindung sind auch die optisch aktiven Formen, die Racemate und die Diastereomerengemische dieser Verbindungen.

Die Erfindung betrifft auch Verfahren zur Herstellung der obigen Verbindungen, Arzneimittel, die solche Verbindungen enthalten, sowie die Verwendung dieser Verbindungen bei der Herstellung von Arzneimitteln.

Die Verbindungen der allgemeinen Formel I, ihre Solvate und ihre Salze hemmen sowohl die durch Thrombin induzierte Gerinnung von Fibrinogen im Blut als auch die durch Thrombin induzierte Aggregation der Blutplättchen. Sie verhindern damit die Entstehung von Gerinnungsthromben und von plättchenreichen Thromben und können bei der Bekämpfung und Verhütung von Krankheiten, wie Thrombose. Apoplexie, Herzinfarkt. Entzündungen und Arteriosklerose, verwendet werden. Ferner haben diese Verbindungen einen Effekt auf Tumorzellen und verhindern die Bildung von Metastasen. Somit können sie als Antitumormittel eingesetzt werden.

Thrombin, das letzte Enzym der Gerinnungskaskade, spaltet Fibrinogen zu Fibrin, das durch den Faktor XIIIa quervernetzt und zu einem unlöslichen Gel wird, das die Matrix für einen Thrombus bildet. Thrombin aktiviert durch Proteolyse seines Rezeptors auf den Blutplättchen die Plättchenaggregation und trägt auf diesem Weg ebenfalls zur Thrombusbildung bei. Bei der Verletzung eines Blutgefäßes sind diese Prozesse notwendig, um eine Blutung zu stoppen. Unter normalen Umständen sind keine meßbaren Thrombin-Konzentrationen im Blutplasma vorhanden. Ansteigen der Thrombinkonzentration kann zur Ausbildung von Thromben und damit zu thromboembolischen Krankheiten führen, die vor allem in den Industriestaaten sehr häufig auftreten.

Thrombin wird im Plasma in Form des Prothrombins bereitgehalten und durch den Faktor Xa aus diesem freigesetzt. Thrombin aktiviert die Faktoren V, VIII und XI, wodurch dann der Faktor X in Xa umgewandelt wird. Thrombin katalysiert dadurch seine eigene Freisetzung, weshalb es zu sehr rasch ansteigenden Thrombin-Konzentrationen kommen kann.

Thrombin-Inhibitoren können deshalb die Freisetzung des Thrombins, die plättcheninduzierte und die plasmatische Blutgerinnung hemmen.

Neben Thrombin existieren noch eine ganze Reihe von Serinproteasen, die Peptidsubstrate neben einer basischen Aminosäure spalten. Um Nebenwirkungen gering zu halten. sollten die Thrombininhibitoren selektiv sein, d. h. sie sollten andere Serinproteasen nur wenig oder gar nicht hemmen. Besonders Trypsin als unspezifischste SerinProtease kann von den verschiedensten Hemmern leicht gehemmt werden. Trypsinhemmung kann zu Pancreas-Stimulation und zu Pancreas-Hypertrophie führen (J.D.Geratz. Am. J. Physiol. 216, (1969) S. 812).

Plasma enthält das Protein Plasminogen, das durch Aktivatoren in Plasmin umgewandelt wird. Plasmin ist ein

proteolytisches Enzym, dessen Aktivität der des Trypsins ähnelt. Es dient zur Auflösung der Thromben, indem es Fibrin abbaut. Hemmung des Plasmins hätte also gerade den gegenteiligen Effekt, den man mit der Hemmung des Thrombins erzielen möchte.

Synthetische Thrombin-Inhibitoren sind schon lange bekannt. Ausgehend vom Fibrinogen, dem natürlichen Substrat des Thrombins, wurden Substanzen des (D)-Phe-Pro-Arg-Typs synthetisiert. Solche Tripeptide ahmen die Aminosäuresequenz vor der Spaltstelle am Fibrinogen nach. Um gute Inhibitoren zu erhalten, wurde die Carboxylatgruppe des Arginins dabei so verändert, daß die Hydroxygruppe des Serin-195 der active site des Thrombins mit ihr reagieren kann. Dies ist beispielsweise dadurch möglich, daß man die Carboxylatgruppe durch die Aldehydfunktion ersetzt. Entsprechende (D)-Phe-Pro-Arginale sind in der Patentanmeldung EP-A-185390 beschrieben.

Zu einem zweiten Typ von Thrombin-Inhibitoren wurde das als Trypsin-Inhibitor bekannte Benzamidin zur Grundlage genommen. Die so erhaltenen Inhibitoren unterscheiden sich von den (D)-Phe-Pro-Arg-Typen nicht nur im chemischen Aufbau, sondern auch in der Art der Inhibierung: das Serin-195 des Thrombins bindet nicht an diese Inibitoren. Dies geht aus Röntgenstruktur-Unteruchungen eindeutig hervor (W. Bode. D. Turk, J. Stürzebecher, Eur. J. Biochem. 193, 175-182 (1990)). Zu dieser zweiten Klasse von Thrombin-Inhibitoren gehört das Na-(2-Naphthylsulfonylglycyl)-4-amidino-(R,S)-phenylalanin-piperidid ("NAPAP", DD 235866).

Überraschend wurde nun gefunden, daß Verbindungen der allgemeinen Formel I, die keine strukturellen Gemeinsamkeiten mit den bekannten Thrombin-Inhibitoren aufweisen. selektive Thrombin-Inhibitoren sind.

Bedeutet in der allgemeinen Formel I $R^1$ eine Arylgruppe, so versteht man darunter die Phenyl- und die Naphthylgruppe. Unter Heteroarylrest für $R^1$ sind mono-, bi- und tricyclische Aromaten mit Heteroatomen wie Stickstoff, Sauerstoff und Schwefel zu verstehen, bevorzugt Furan, Thiophen, Pyrrol, Oxazol, Isoxazol, Thiazol, Isothiazol, Imidazol, Pyrazol. Triazol. Tetrazol, Pyridin, Pyrazin, Pyrimidin, Pyridazin, Triazin, Tetrazin, Benzothiophen, Dibenzothiophen, Benzimidazol, Carbazol, Benzofuran, Benzofurazan, Benzo-2,1,3-thiadiazol, Chinolin, Isochinolin, Chinazolin.

Halogene als Substituenten der Aryl- oder Heteroarylreste bedeuten Chlor-, Brom- und Iod-atome, bevorzugt aber Fluoratome.

Alkoxycarbonylgruppen als Substituenten der Aryl- oder Heteroarylreste enthalten geradkettige oder verzweigte Alkylketten mit 1 bis 6 Kohlenstoffatomen. Bevorzugt sind die Methoxycarbonyl- und die Ethoxycarbonylgruppe.

Phenylalkoxycarbonylgruppen als Substituenten der Aryl- oder Heteroarylreste enthalten eine mit einer $C_1$-$C_6$-Alkylkette verknüpfte Phenylgruppe. Bevorzugt ist dabei die Benzyloxycarbonylgruppe.

Alkylgruppen als Substituenten der Aryl- oder Heteroarylreste sind geradkettig oder verzweigt und enthalten 1 bis 6 Kohlenstoffatome. Bevorzugt sind die Methyl-, Ethyl-, Propyl-. Butyl-, Pentyl- und die Hexylgruppe.

Alkoxygruppen als Substituenten der Aryl- oder Heteroarylreste enthalten 1 bis 6 Kohlenstoffatome und sind geradkettig oder verzweigt. Bevorzugt sind die Methoxy-, Ethoxy-, *n*-Propyloxy-, *i*-Propyloxy-, *n*-Butyloxy-, *i*-Butyloxy-, *tert.*-Butyloxy-, Pentyloxy- und die Hexyloxygruppe.

Bedeutet in der allgemeinen Formel I $R^1$ eine mit einem Alkenyloxyrest substituierte Aryl- oder Heteroarylgruppe, so sind darunter geradkettige oder verzweigte Reste mit 3 bis 6 Kohlenstoffatomen zu verstehen, vorzugsweise die Allyloxygruppe.

Bedeutet in der allgemeinen Formel I $R^1$ eine mit einem Alkinyloxyrest substituierte Aryl- oder Heteroarylgruppe, so sind darunter geradkettige oder verzweigte Reste mit 1 bis 6 Kohlenstoffatomen zu verstehen, vorzugsweise die Propargyloxygruppe.

Bedeutet in der allgemeinen Formel I $R^1$ eine mit einem Aralkyloxyrest substituierte Aryl- oder Heteroarylgruppe, so ist darunter bevorzugt der Benzyloxyrest zu verstehen.

Bedeutet in der allgemeinen Formel I $R^1$ eine mit einem Alkylthio-. Alkylsulfinyl- oder Alkylsulfonylrest substituierte Aryl- oder Heteroarylgruppe. so sind darunter geradkettige oder verzweigte Reste mit 1 bis 6 Kohlenstoffatomen zu verstehen, vorzugsweise die Methylthio, Methylsulfinyl- und die Methylsulfonylgruppe.

Bedeutet in der allgemeinen Formel I $R^1$ eine mit einem Alkylamino- oder Dialkylaminorest substituierte Aryl- oder Heteroarylgruppe, so sind darunter geradkettige oder verzweigte Reste mit 1 bis 6 Kohlenstoffatomen zu verstehen, vorzugsweise die Methylamino, Dimethylamino- und die Diethylaminogruppe.

Bedeutet in der allgemeinen Formel I $R^1$ einen Aralkylaminorest oder einen Di-aralkylaminorest, so sind die Benzylaminogruppe und die Bis(benzyl)aminogruppe besonders bevorzugt.

Bedeutet in der allgemeinen Formel I $R^1$ eine mit einem Alkylsulfonylaminorest substituierte Aryl- oder Heteroarylgruppe, so sind darunter geradkettige oder verzweigte Reste mit 1 bis 6 Kohlenstoffatomen zu verstehen, vorzugsweise die Methylsulfonylaminogruppe.

Bedeutet in der allgemeinen Formel I $R^1$ eine mit einem Alkylcarbonylaminorest substituierte Aryl- oder Heteroarylgruppe, so sind darunter geradkettige oder verzweigte Reste mit 1 bis 6 Kohlenstoffatomen zu verstehen, vorzugsweise die Acetylaminogruppe.

Bedeutet in der allgemeinen Formel I $R^1$ eine mit einem Alkylaminocarbonyl- oder Dialkylaminocarbonylrest substituierte Aryl- oder Heteroarylgruppe, so sind darunter geradkettige oder verzweigte Reste mit 1 bis 6 Kohlenstoffatomen zu verstehen, vorzugsweise die Methylaminocarbonyl-, Dimethylaminocarbonyl- und die Diethylaminocarbonylgruppe.

Bedeutet in der allgemeinen Formel I $R^1$ eine mit einem Alkoxycarbonylalkyloxyrest substituierte Aryl- oder Heteroarylgruppe, so ist dabei die Ethoxycarbonylmethyloxygruppe besonders bevorzugt.

In der allgemeinen Formel I versteht man unter den Alkylgruppen für $R^2$ und $R^3$ geradkettige oder verzweigte Reste mit 1 bis 6 Kohlenstoffatomen, wie die Methyl-, Ethyl-, Propyl-, Butyl-, Pentyl- und die Hexylgruppe.

In der allgemeinen Formel I versteht man unter den Alkylengruppen für A geradkettige oder verzweigte Reste mit 1 bis 6 Kohlenstoffatomen, wie die Methylen-, Ethylen-, Propylen-, Butylen-, Pentylen- und die Hexylengruppe.

$R^1$ ist insbesondere eine unsubstituierte oder ein- oder mehrfach durch $C_1$-$C_6$-Alkoxygruppen (wie z.B. Methoxy, Propyloxy, Butoxy und Hexyloxy) substituierte Phenylgruppe,

$R^2$ und $R^3$ sind insbesondere gleich und $C_1$-$C_6$-Alkylgruppen (wie z.B. die Methylgruppe).

A ist insbesondere eine $C_1$-$C_6$-Alkylengruppe (wie z.B. die Methylengruppe).

Bevorzugt sind Verbindungen der allgemeinen Formel I.

(I)

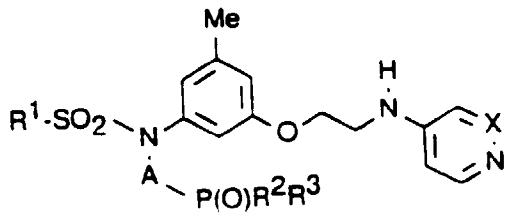

in der

| | |
|---|---|
| $R^1$ | eine unsubstituierte oder durch eine Methoxy-, Ethoxy-, Propyloxy-, Isopropyloxy-, Butoxy- oder Hexyloxygruppe substituierte Phenylgruppe bedeutet, |
| $R^2$ und $R^3$ | gleich sind und Methylgruppen bedeuten, |
| A | die Methylengruppe bedeutet |
| X | die -CH-Gruppe und das Stickstoffatom bedeutet. |

Die Herstellung von Verbindungen der allgemeinen Formel I geschieht nach an sich bekannten Verfahren.

Man hydriert die Verbindungen der allgemeinen Formel II,

II

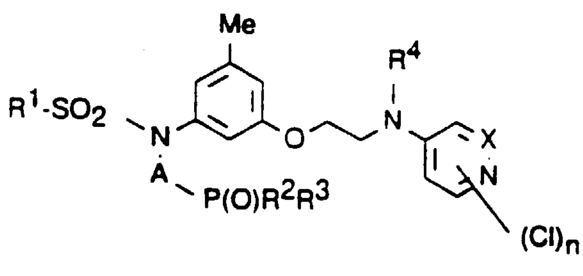

in der $R^1$, $R^2$, $R^3$, A und X die oben angegebenen Bedeutungen besitzen. $R^4$ bedeutet das Wasserstoffatom oder die Benzylgruppe. Für den Fall, das X die -CH-Gruppe bedeutet, ist n = 4. Für den Fall, daß X das Stickstoffatom bedeutet, ist n = 2. Die Hydrierung geschieht in einem inerten Lösungsmittel wie Methanol oder Ethanol in Gegenwart eines Katalysators wie Palladium auf Kohle oder Raney-Nickel und in Gegenwart einer Base wie N-Methylmorpholin, Triethylamin, Kaliumcarbonat, Natriumbicarbonat oder Natrium-methylat, vorzugsweise bei Normaldruck und Raumtemperatur. Die Hydrierung gelingt auch in Abwesenheit einer Base. Für den Fall, daß $R^4$ die Benzylgruppe bedeutet, kann

diese gewünschtenfalls auch vor der Hydrierung entfernt werden. Dies gelingt durch Umsetzung mit einer starken Säure wie Trifluoressigsäure in Gegenwart von Mesitylen, Anisol oder Thioanisol.

Die Verbindungen der allgemeinen Formel II stellt man her durch Umsetzung der Verbindungen der allgemeinen Formel III mit den Phosphanoxiden der allgemeinen Formel IV.

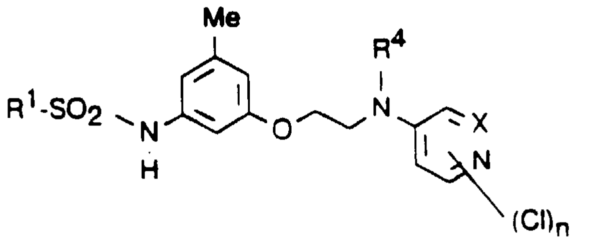

+

Hal-A-P(O)$R^2R^3$

(III)

(IV)

In der allgemeinen Formel III haben $R^1$, $R^4$ und n die oben angegebenen Bedeutungen. In der allgemeinen Formel IV haben $R^2$, $R^3$ und A die oben angegebenen Bedeutungen und Hal bedeutet ein Halogenatom, vorzugsweise das Chloratom. Die Umsetzung geschieht in einem inerten Lösungsmittel oder ohne Lösungsmittel in einer geschmolzenen Mischung der Verbindungen III und IV und einer Base wie Natriumhydrid oder Kaliumcarbonat bei Temperaturen zwischen 100 und 200 °C.

Die Verbindungen der allgemeinen Formel IV sind literaturbekannt oder käuflich.

Eine bevorzugte Methode zur Herstellung von Verbindungen der allgemeinen Formel III besteht in der Umsetzung von Aminen der allgemeinen Formel V,

V

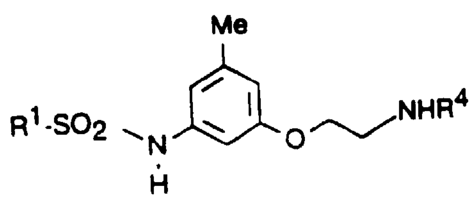

in der $R^1$ und $R^4$ die oben angegebenen Bedeutungen haben, mit Pentachlorpyridin oder 4-Nitrotetrachlorpyridin, wobei Verbindungen der allgemeinen Formel III entstehen, in denen X die -CH-Gruppe ist, oder mit 3,4,5-Trichlorpyridazin, wobei die Verbindungen der allgemeinen Formel III entstehen, in denen X das Stickstoffatom bedeutet. Diese Umsetzung führt man in einem inerten Lösungsmittel wie Ethanol, Toluol. Tetrahydrofuran oder Dimethylformamid bei Temperaturen zwischen 0 und 100 °C in Gegenwart einer Base wie Triethylamin, N-Methylmorpholin oder Kaliumcarbonat durch.

Die Verbindungen der allgemeinen Formel V erhält man aus den Verbindungen der allgemeinen Formel VI,

VI

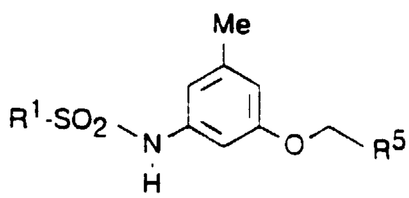

in der $R^1$ die oben angegebenen Bedeutungen hat und $R^5$ die Nitrilgruppe -CN, die Amidgruppe $-CONH_2$ oder die Phthalimidomethylgruppe ist. Die Freisetzung der Aminomethylfunktion aus dem Rest $R^5$ geschieht in an sich bekannter Weise. Für den Fall, daß $R^5$ die Nitrilgruppe bedeutet, geschieht dies durch Hydrierung in Gegenwart eine Katalysators wie Raney-Nickel oder Palladium auf Kohle. oder durch Reduktion mit Lithiumaluminiumhydrid oder Lithiumborhydrid in Gegenwart von Trimethylsilylchlorid. Für den Fall, daß $R^5$ die Amidgruppe bedeutet, geschieht dies durch Reduktion mit Lithiumaluminiumhydrid oder durch Lithiumborhydrid in Gegenwart von Trimethylsilylchlorid. Für den Fall, daß $R^5$ die Phthalimidomethylgruppe bedeutet, geschieht dies durch eine Säure wie Salzsäure, oder durch eine Base wie Natronlauge oder Kalilauge oder durch die Einwirkung von Hydrazinhydrat. Dabei entstehen zunächst die Verbindungen der allgemeinen Formel V, in denen $R^4$ das Wasserstoffatom bedeutet. Gewünschtenfalls können daraus die Verbindungen der allgemeinen Formel V, in denen $R^4$ die Benzylgruppe bedeutet, hergestellt werden. Dies geschieht durch reduktive Aminierung, indem man die Amine der allgemeinen Formel V mit Benzaldehyd in einem inerten Lösungsmittel wie Toluol in Gegenwart katalytischer Mengen einer Säure wie Toluol-4-sulfonsäure und anschließend mit Natriumborhydrid umsetzt.

Die Verbindungen der allgemeinen Formel VI erhält man durch Umsetzung von verbindungen der allgemeinen Formel VII

VII

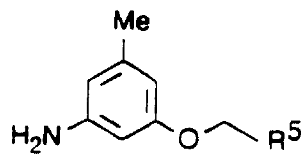

in denen $R^5$ die oben angegebenen Bedeutungen hat, mit den Sulfonsäurechloriden $R^1$-$SO_2Cl$, wobei $R^1$ die oben angegebenen Bedeutungen hat. Die Umsetzung erfolgt zweckmäßig unter Zusatz eines saurebindenden Mittels, wie z.B. Alkaliacetat. Alkalihydroxid, Calciumoxid, Calciumcarbonat, Magnesiumcarbonat oder mit organischen Basen wie Pyridin, Triethylamin, N-Methylmorpholin oder Di-isopropylethylamin, wobei als inerte Lösungsmittel z.B. Ether, Methylenchlorid, Dioxan, Toluol oder ein Überschuß des tertiären Amins dienen. Beim Einsatz anorganischer Säurebinder verwendet man als Reaktionsmedium z.B. Wasser oder wäßriges Ethanol.

Die Sulfonsäurechloride $R^1$-$SO_2Cl$ sind käuflich oder können nach literaturbekannten Verfahren hergestellt werden (Methoden der Organischen Chemie (Houben-Weyl), Thieme Verlag, Stuttgart 1955, S.429: F. Muth, Aromatische Sulfonsäuren).

Die Verbindungen der allgemeinen Formel VII erhält man aus den Verbindungen der allgemeinen Formel VIII,

VIII

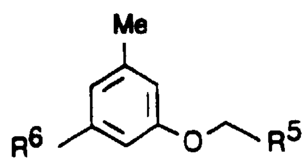

in der $R^5$ die oben genannten Bedeutungen hat und $R^6$ eine geschützte Aminogruppe ist. Unter geschützten Aminogruppen versteht man bevorzugt die Benzyloxycarbonylaminogruppe -NH-$CO_2CH_2Ph$, die *tert.*Butyloxycarbonylaminogruppe -NH-$CO_2$-*t.*Bu, oder die Phthalimidogruppe. Die Freisetzung der Aminogruppe oder der Hydroxygruppe geschieht in an sich bekannter Weise. Die Benzyloxycarbonylaminogruppe wandelt man durch Hydrierung in Gegenwart eines Katalysators wie Raney-Nickel oder Palladium auf Kohle, oder durch eine Säure wie konzentrierte Ameisensaure. Salzsäure oder mit Bromwasserstoff in Eisessig in die freie Aminogruppe um. Die *tert.*Butyloxycarbonylaminogruppe wandelt man durch eine Säure wie Salzsäure in Dioxan. Ameisensäure oder Trifluoressigsäure in die Aminogruppe um. Die Phthalimidogruppe wandelt man durch eine Säure wie Salzsäure, oder durch eine Base wie Natronlauge oder Kalilauge oder durch die Einwirkung von Hydrazinhydrat in die Aminogruppe um.

Die Verbindungen der allgemeinen Formel VIII stellt man her durch Umsetzung der Phenole der allgemeinen Formel IX mit den Verbindungen der allgemeinen Formel X.

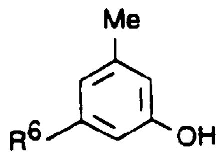

(IX)

+

$R^7-CH_2-R^8$

(X)

In den Verbindungen der allgemeinen Formel IX bedeutet $R^6$ eine geschützte Aminogruppe wie die Benzyloxycarbonylaminogruppe $-NH-CO_2CH_2Ph$, die *tert.*Butyloxycarbonylaminogruppe $-NH-CO_2$-*t*.Bu, oder die Phthalimidogruppe. In den Verbindungen der allgemeinen Formel X hat $R^8$ die gleichen Bedeutungen wie $R^5$ (Nitril-, Amid- oder Phthalimidomethylgruppe) und die Carbonestergruppe. $R^7$ bedeutet ein Chlor-, Brom- oder Iod-Atom oder eine Hydroxy- oder Arylsulfonyloxygruppe. Ist $R^7$ ein Chlor-, Brom- oder Iod-Atom oder eine Arylsulfonyloxygruppe, geschieht die Umsetzung vorzugsweise in einem Lösungsmittel wie Aceton, Ether, Toluol oder Dimethylformamid bei Temperaturen zwischen -30°C und 100°C, vorzugsweise in Gegenwart einer Base wie Natriumhydrid oder Kaliumcarbonat. Ist $R^7$ die Hydroxygruppe, geschieht die Umsetzung in einem inerten Lösungsmittel in Gegenwart von Diazodicarbonsäure-diethylester oder -dipiperidid und Triphenylphosphin. Bedeutet in der allgemeinen Formel IX $R^8$ die Carbonester-Gruppe, so wird diese nun verseift, vorzugsweise durch Kaliumhydroxid in Methanol und dann mit Ammoniak in die Amidgruppe $CONH_2$ überführt. Diese Umwandlung kann mit Hilfe von $CH_3Al(Cl)NH_2$, das man aus Trimethylaluminium und Ammoniumchlorid herstellt, auch direkt ohne vorherige Verseifung erfolgen.

Die Verbindungen der allgemeinen Formel IX stellt man her durch Umsetzung von 3-Amino-5-methyl-phenol (F. Wessely, H. Eibel, G. Friedrich. Monatshefte Chem. 83, 24 - 30 (1952)) mit Anhydriden wie Phthalsäureanhydrid oder BOC-Anhydrid (tert.Butyloxycarbonsäure-anhydrid) oder mit Benzyloxycarbonylchlorid. Die Verbindungen der allgemeinen Formel X sind käuflich.

Eine weitere bevorzugte Methode zur Herstellung von Verbindungen der allgemeinen Formel III besteht in der Umsetzung von Verbindungen der allgemeinen Formel XI

XI

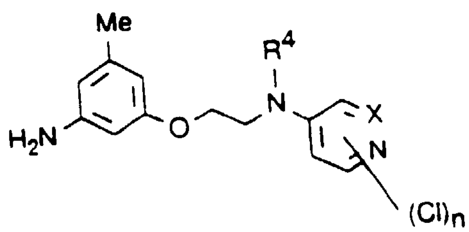

in der $R^4$, X und n die oben angegebenen Bedeutungen haben, mit den Sulfonsäurechloriden $R^1SO_2Cl$, wobei $R^1$ die oben angegebenen Bedeutungen hat. Die Umsetzung erfolgt zweckmäßig unter Zusatz eines säurebindenden Mittels, wie z.B. Alkaliacetat. Alkalihydroxid, Calciumoxid, Calciumcarbonat, Magnesiumcarbonat oder mit organischen Basen wie Pyridin, Triethylamin, N-Methylmorpholin oder Di-isopropylethylamin, wobei als inerte Lösungsmittel z.B. Ether, Methylenchlorid, Dioxan, Toluol oder ein Überschuß des tertiären Amins dienen. Beim Einsatz anorganischer Säurebinder verwendet man als Reaktionsmedium z.B. Wasser, wäßriges Ethanol.

Die Verbindungen der allgemeinen Formel XI stellt man her aus den Verbindungen der allgemeinen Formel XII,

XII

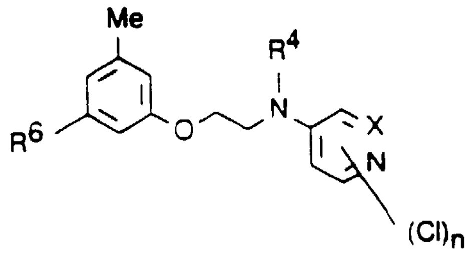

in der $R^4$, $R^6$, X und n die oben angegebenen Bedeutungen haben. Die Freisetzung der Aminogruppe geschieht in an sich bekannter Weise. Die Benzyloxycarbonylaminogruppe wandelt man durch Hydrierung in Gegenwart eines Katalysators wie Raney-Nickel oder Palladium auf Kohle, oder durch eine Säure wie konzentrierte Ameisensäure. Salzsäure oder mit Bromwasserstoff in Eisessig in die freie Aminogruppe um. Die *tert.*Butyloxycarbonylaminogruppe wandelt man durch eine Säure wie Salzsäure in Dioxan. Ameisensäure oder Trifluoressigsäure in die Aminogruppe um. Die Phthalimidogruppe wandelt man durch eine Säure wie Salzsaure, oder durch eine Base wie Natronlauge oder Kalilauge oder durch die Einwirkung von Hydrazinhydrat in die Aminogruppe um.

Die Verbindungen der allgemeinen Formel XII erhält man durch Umsetzung der Verbindungen der allgemeinen Formel IX mit den Verbindungen der allgemeinen Formel XIII.

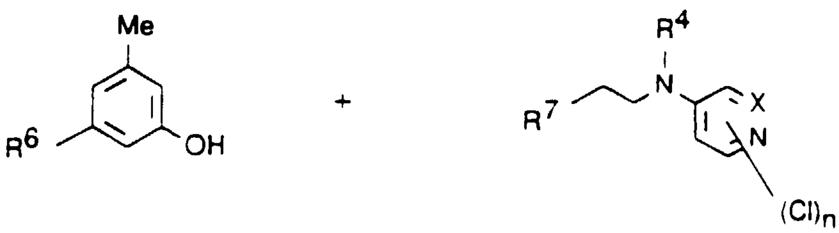

+

(IX) (XIII)

In der allgemeinen Formel IX hat $R^6$ die oben angegebenen Bedeutungen. In der allgemeinen Formel XIII haben $R^4$, $R^7$, X und n die oben angegebenen Bedeutungen. Ist $R^7$ ein Chlor-. Brom- oder Iod-Atom oder eine Arylsulfonyloxygruppe, geschieht die Umsetzung vorzugsweise in einem Lösungsmittel wie Aceton. Ether. Toluol oder Dimethylformamid bei Temperaturen zwischen -30°C und 100°C. vorzugsweise in Gegenwart einer Base wie Natriumhydrid oder Kaliumcarbonat. Ist $R^7$ die Hydroxygruppe, geschieht die Umsetzung in einem inerten Lösungsmittel in Gegenwart von Diazodicarbonsäure-diethylester oder -dipiperidid und Triphenylphosphin.

Die Verbindungen der allgemeinen Formel XIII erhält man durch Umsetzung von Ethanolamin mit Pentachlorpyridin oder 4-Nitrotetrachlorpyridin (falls X die -CH-Gruppe bedeutet) oder mit 3,4,5-Trichlorpyridazin (falls X das Stickstoffatom bedeutet). Diese Umsetzung führt man in einem inerten Lösungsmittel wie Ethanol, Toluol. Tetrahydrofuran oder Dimethylformamid bei Temperaturen zwischen 0 und 100 °C in Gegenwart einer Base wie Triethylamin. N-Methylmorpholin oder Kaliumcarbonat durch. Dabei entstehen zunächst diejenigen Verbindungen der allgemeinen Formel XIII, in denen $R^7$ die Hydroxygruppe und $R^4$ das Wasserstoffatom bedeutet. Gewünschtenfalls kann man nun $R^4$ in die Benzylgruppe umwandeln. indem man die Hydroxygruppe acetyliert, dann mit Benzylbromid oder Benzylchlorid umsetzt und die Acetylgruppe wieder abspaltet. Die Hydroxygruppe läßt sich nun gewünschtenfalls in die Toluol-4-sulfonyloxygruppe oder in ein Halogenatom umwandeln. Dies geschieht durch Umsetzung mit Toluol-4-sulfonylchlorid, Thionylchlorid oder -bromid.

Beispiele von physiologisch verwendbaren Salzen der Verbindungen der Formel I sind Salze mit physiologisch verträglichen Mineralsäuren, wie Salzsäure, Schwefelsäure, schweflige Säure oder Phosphorsäure. oder mit organischen Säuren, wie Methansulfonsäure, p-Toluolsulfonsaure, Essigsäure, Trifluoressigsäure. Zitronensäure. Fumarsäure, Maleinsäure, Weinsäure, Bernsteinsäure oder Salicylsäure. Die Verbindungen der Formel I mit freier Carboxygruppe können auch Salze mit physiologisch verträglichen Basen bilden. Beispiele solcher Salze sind Alkalimetall-, Erdalkalimetall-, Ammonium- und Alkylammoniumsalze, wie das Natrium-, Kalium-. Calcium- oder Tetra- methylammoniumsalz.

Die Verbindungen der Formel I können solvatisiert, insbesondere hydratisiert sein. Die Hydratisierung kann im Zuge der Herstellung erfolgen oder allmählich als Folge hygroskopischer Eigenschaften einer zunächst wasserfreien Verbindung der Formel I auftreten.

Zu reinen Enantiomeren der Verbindungen der Formel I kommt man entweder durch Racematspaltung (über Salzbildung mit optisch aktiven Säuren oder Basen), oder indem man in die Synthese optisch aktive Ausgangsstoffe einsetzt.

Zur Herstellung von Arzneimitteln werden die Substanzen der allgemeinen Formel I mit geeigneten pharmazeutischen Trägersubstanzen. Aroma-. Geschmacks- und Farbstoffen gemischt und beispielsweise als Tabletten oder Dragees ausgeformt oder unter Zugabe entsprechender Hilfsstoffe in Wasser oder Öl, z.B. in Olivenöl, suspendiert oder gelöst.

Die Substanzen der allgemeinen Formel I und ihre Salze können in flüssiger oder fester Form enteral oder parenteral appliziert werden. Als Injektionsmedium kommt vorzugsweise Wasser zur Anwendung, welches die bei Injektionslösungen üblichen Zusatze wie Stabilisierungsmittel. Lösungsvermittler oder Puffer enthält. Derartige Zusätze sind z.B. Tartrat- und Citratpuffer, Komplexbildner (wie Ethylendiamintetraessigsäure und deren untoxische Salze) und hochmolekulare Polymere wie flüssiges Polyethylenoxid zur Viskositätsregulierung. Feste Trägerstoffe sind z.B. Stärke, Lactose, Mannit, Methylcellulose, Talcum, hochdisperse Kieselsäuren, hochmolekulare Fettsäuren (wie Stearinsäure), tierische und pflanzliche Fette und feste hochmolekulare Polymere (wie Polyethylenglykole). Für orale Applikation geeignete Zubereitungen können gewünschtenfalls Geschmacks- und Süßstoffe enthalten.

Die Verbindungen werden üblicherweise in Mengen von 10-1500 mg pro Tag bezogen auf 75 kg Körpergewicht appliziert. Bevorzugt ist es, 2-3 mal pro Tag 1-2 Tabletten mit einem Wirkstoffgehalt von 5-500 mg zu verabreichen. Die Tabletten können auch retardiert sein, wodurch nur noch einmal pro Tag 1-2 Tabletten mit 20-700 mg Wirkstoff gegeben werden müssen. Der Wirkstoff kann auch durch Injektion 1-8 mal pro Tag oder durch Dauerinfusion gegeben werden, wobei 50-2000 mg pro Tag normalerweise ausreichen.

Bevorzugt im Sinne der Erfindung sind außer den in den Beispielen genannten Verbindungen die folgenden:

1. N-(Dimethyloxophosphinylmethyl)-N-{3-methyl-5-[2-(pyridin-4-ylamino)-ethoxy]-phenyl}-2-ethoxy-benzolsulfonamid

2. N-(Dimethyloxophosphinylmethyl)-N-{3-methyl-5-[2-(pyridin-4-ylamino)-ethoxy]-phenyl}-2-propyloxy-benzolsulfonamid

3. N-(Dimethyloxophosphinylmethyl)-N-{3-methyl-5-[2-(pyridin-4-ylamino)-ethoxy]-phenyl}-2-(2-propyl-oxy)-benzolsulfonamid

5. N-(Dimethyloxophosphinylmethyl)-N-{3-methyl-5-[2-(pyridin-4-ylamino)-ethoxy]-phenyl}-2-pentyloxy-benzolsulfonamid

## Beispiel 1

N-(Dimethyloxophosphinylmethyl)-N-{3-methyl-5-[2-(pyridin-4-ylamino)-ethoxy]-phenyl}-benzolsulfonamid

a) 36.0 g (292 mmol) 3-Hydroxy-5-methyl-anilin (F. Wessely, H. Eibel, G. Friedrich, Monatshefte Chem. 83, 24 - 30, (1952)) und 73.5 g (496 mmol) Phthalsäureanhydrid erhitzte man in 280 mL Eisessig 2 h unter Rückfluß zum Sieden. Man gab Wasser zu, erhitzte kurz, ließ abkühlen und fitrierte. Man erhielt 59.6 g (80%) 2-(3-Hydroxy-5-methyl-phenyl)-isoindol-1,3-dion mit dem Fp. 174 - 175 °C.

b) Zu einer Suspension von 230 g (915 mmol) Pentachlorpyridin in 3.2 L trockenem Dimethylformamid tropfte man unter Eiskühlung innerhalb von 15 min 138 mL (2.5 mol) Ethanolamin, rührte 30 min unter Eiskühlung und 3 h bei Raumtemperatur. Man entfernte das Lösungsmittel zum größten Teil, goß den Rest auf 4 L Eiswasser, filtierte und kristalisierte den Rückstand aus 800 mL Dichlormethan um. Man erhielt 172 g (69%) 2-(2,3,5,6-tetrachlor-pyridin-4-ylamino)-ethanol mit dem Fp. 128 - 130 °C.

c) Zu einer Suspension von 105 g (380 mmol) 2-(2,3,5,6-tetrachlor-pyridin-4-ylamino)-ethanol in 860 mL Eisessig tropfte man eine Lösung von 34.4. mL (490 mmol) Acetylchlorid in 250 mL Eisessig. Die Temperatur stieg dabei auf 40 °C Nach 2 h goß man auf Eiswasser, filtrierte, wusch mit Wasser, löste den Rückstand in 500 mL Essigester trocknete über Natriumsulfat. filtrierte und entfernte das Losungsmittel i. Vak. Man erhielt 112 g (93%) Essigsäure-2-(2,3,5,6-tetrachlor-pyridin-4-ylamino)-ethylester, das man ohne weitere Reinigung umsetzte. MS (m/e) = 316.

d) Zu einer Suspension von 11.5 g (455 mmol) Natriumhydrid (95%ig) in 200 mL Dimethylformamid tropfte man bei 10 °C innerhalb von 10 min eine Lösung von 111 g (350 mmol) Essigsäure-2-(2,3,5,6-tetrachlor-pyridin-4-ylamino)-ethylester in 500 mL Dimethylformamid. Nach 1 h tropfte man innerhalb von 10 min unter Eiskühlung 54 mL (455 mmol) Benzylbromid zu. Nach 2 h bei Raumtemperatur gab man 50 mL Isopropanol zu und goß nach 30 min auf 7 L Eiswasser. Man filtrierte, löste den Rückstand in Essigester. trocknete über Natriumsulfat, filtrierte und entfernte das Lösungsmittel i. Vak. Man digerierte mit Ether und erhielt 73.8 g (52%) Essigsäure-2-[benzyl-(2,3,5,6-tetrachlor-pyridin-4-yl)-amino]-ethylester mit dem Fp. 98 - 100 °C.

e) 187 g (436 mmol) Essigsaure-2-[benzyl-(2,3,5,6-tetrachlor-pyridin-4-yl)-amino]-ethylester, 850 mL Dimethylformamid, 2.1 L Ethanol und 680 mL 2N Natronlauge rührte man 2 h bei Raumtemperatur, entfernte das Lösungsmittel weitgehend i. Vak., löste den Rückstand in 1 L Essigester, extrahierte 2 mal mit 3 L Wasser, trocknete die organische Phase über Natriumsulfat, filtrierte, entfernte das Lösungsmittel i. Vak., digerierte den Rückstand in wenig Diisopropylether, gab Isohexan zu und ließ kristallisieren. Man filtrierte, wusch mit Isohexan und erhielt 140 g (84%) 2-[Benzyl-(2,3,5,6-tetrachlor-pyridin-4-yl)-amino]-ethanol mit dem Fp. 79 - 81 °C.

f) Zu 50.4 g (138 mmol) 2-[Benzyl-(2,3,5,6)-tetrachlor-pyridin-4-yl)-amino]-ethanol und 34.4 mL Triethylamin in 600 mL trockenem Dichlormethan tropfte man unter Eiskühlung 31.5 g (165 mmol) Toluol-4-sulfonylchlorid in 100 mL Dichlormethan und bewahrte 14 h bei 5 °C auf. Man extrahierte mit Wasser, trocknete die organische Phase mit Natriumsulfat, filtrierte, entfernte das Lösungsmittel i. Vak., digerierte den Rückstand mit Methanol, filtrierte und erhielt 58.0 g (81%) Toluol-4-sulfonsäure-2-[benzyl-(2,3,5,6-tetrachlor-pyridin-4-yl)-amino]-ethylester mit dem Fp. 114 - 116 °C.

g) Zu 3.8 g (150 mmol) Natriumhydrid (95%ig) in 400 mL Dimethylformamid tropfte man unter Eiskühlung eine Lösung von 24.3 g (96 mmol) 2-(3-Hydroxy-5-methyl-phenyl)-isoindol-1,3-dion in 200 mL Dimethylformamid. Nach 30 min tropfte man diese Lösung zu 50 g (96 mmol) Toluol-4-sulfonsaure-2-[benzyl-(2,3,5,6-tetrachlor-pyridin-4-yl)-amino]-ethylester in 360 mL Dimethylformamid. Nach 1 h goß man auf Eiswasser, extrahierte dreimal mit Essigester, extrahierte die organische Phase fünfmal mit Wasser. trocknete die organische Phase über Natriumsulfat, filtrierte, entfernte das Lösungsmittel i. Vak. und erhielt 50.4 g eines öligen Rückstandes, den man über eine Kieselgelsäule (Laufmittel Isohexan Essigester = 9:1 bis 7:3) reinigte. Man erhielt 19.0 g (33%) 2-(3-{2-[Benzyl-(2,3,5,6-tetrachlor-pyridin-4-yl)-amino]-ethoxy}-5-methyl-phenyl)-isoindol-1,3-dion mit dem Fp. 142 - 143 °C.

h) 61.4 g (100 mmol) 2-(3-{2-[Benzyl-(2,3,5,6-tetrachlor-pyridin-4-yl)-amino]-ethoxy}-5-methyl-phenyl)-isoindol-1,3-dion und 7.2 mL (150 mmol) Hydrazinhydrat in 320 mL Dichlormethan und 160 mL Ethanol rührte man 3 d bei Raumtemperatur. Unter Eiskühlung gab man 40 mL konzentrierte Salzsäure zu. verdünnte den Kristallbrei mit Ethanol. rührte 1 h. entfernte das Lösungsmittel i. Vak., suspendierte den Rückstand in 2 N Natronlauge, gab Dichlormethan zu, rührte 30 min., filtrierte, trennte die organische Phase ab. extrahierte die wäßrige Phase mit Dichlormethan. wusch die vereinigten organischen Phasen mit Wasser, entfernte das Lösungsmittel i. Vak., digerierte den Rückstand mit Methanol und erhielt 37.6 g (78%) [2-(3-Amino-5-methyl-phenoxy)-ethyl]-benzyl-(2,3,5,6-tetrachlor-pyridin-4-yl)-amin mit dem Fp. 92 - 93 °C.

i) Zu 11.5 g (25.5 mmol) [2-(3-Amino-5-methyl-phenoxy)-ethyl]-benzyl-(2,3,5,6-tetrachlor-pyridin-4-yl)-amin in 55 mL Pyridin tropfte man unter Eiskühlung 3.5 mL (27 mmol) Benzolsulfonylchlorid. Nach 1 h goß man aufEis, das mit 130 mL 6 N Salzsäure versetzt war, extrahierte mit Essigester, trocknete über Natriumsulfat, filtrierte, entfernte das Lösungsmittel i. Vak., digerierte den Rückstand mit 100 mL Ether/Diisopropylether (1:1) und erhielt 13.7 g (91%) N-(3-{2-[Benzyl-(2,3,5,6-tetrachlor-pyridin-4-yl)-amino]-ethoxy}-5-methyl-phenyl)-benzolsulfonamid mit dem Fp. 147 - 149 °C.

j) 1.0 g N-(3-{2-[Benzyl-(2,3,5,6-tetrachlor-pyridin-4-yl)-amino]-ethoxy}-5-methyl-phenyl)-benzolsulfonamid. 0.62 g Chlormethyl-dimethyl-phosphanoxid und 0.68 g Kaliumcarbonat rührte man 20 min bei 150 - 160 °C, extrahierte mit Methanol, filtrierte, entfernte das Lösungsmittel i. Vak. digerierte den Rückstand mit einer Mischung aus Essigester und Methanol (9:1), filtrierte, reinigte über eine Kieselgelsäule (Laufmittel Essigester / Methanol = 9:1), entfernte das Lösungsmittel i. Vak und erhielt 1.0 g (N-(3-{2-[Benzyl-(2,3,5,6-tetrachlor-pyridin-4-yl)-amino]-ethoxy}-5-methyl-phenyl)-N-(dimethyloxophosphinyl-methyl)benzolsulfonamid als amorphe Masse. MS (m/e) = 701.

k) 1.0 g (1.4 mmol) N-(3-{2-[Benzyl-(2,3,5,6-tetrachlor-pyridin-4-yl)-amino]-ethoxy}-5-methyl-phenyl)-N-(dimethyloxophosphinyl-methyl)-benzolsulfonamid, 1,15 mL 1,3,5-Trimethylbenzol und 8 mL Trifluoressigsäure rührte man 12 h bei Raumtemperatur, goß auf 150 mL Wasser, stellte mit konzentrierter wäßriger Ammoniaklösung alkalisch,

filtrierte, wusch mit Wasser und Ether, löste den Rückstand in Essigester, trocknete uber Natriumsulfat, behandelte mit Kieselgur, filtrierte, entfernte das Lösungsmittel i. Vak., nahm den Rückstand in 3 mL Essigester auf, versetzte mit 30 mL Ether und ließ kristallisieren. Man filtrierte und erhielt 0.5 g (58%) N-(Dimethyloxophosphinyl-methyl)-N-{3-methyl-5-[2-(2,3,5,6)-tetrachlor-pyridin-4-ylamino)-ethoxy]-phenyl}-benzolsulfonamid mit dem Fp. 152 - 154 °C.

l) 0.2 g (0.33 mmol) N-(Dimethyloxophosphinyl-methyl)-N-{3-methyl-5-[2-(2,3,5,6)-tetrachlor-pyridin-4-ylamino)-ethoxy]-phenyl}-benzolsulfonamid in 10 mL Methanol hydrierte man in Gegenwart von 0.28 mL Triethylamin und 0.1 g 10% Palladium auf Kohle bei Raumtemperatur und Normaldruck. Nach 18 h fitrierte man, löste den Rückstand in 3 mL Ethanol, gab 30 mL Ether zu, filtrierte, wusch mit ether und erheilt 0.12 g (77%) der Titelverbindung mit dem Fp. 228 - 232 °C.

**Beispiel 2**

N-(Dimethyloxophosphinylmethyl)-N-{3-methyl-5-{2-(pyridin-4-ylamino)-ethoxy]-phenyl}-2-methoxy-benzolsulfonamid

erhielt man in 24% Ausbeute analog dem Bsp. 1, wobei man in der Stufe 1i) 2-Methoxy-benzolsulfonylchlorid an Stelle von Benzolsulfonylchlorid einsetzte. Amorph. MS (m/e)= 503.

**Beispiel 3**

N-(Dimethyloxophosphinylmethyl)-N-{3-methyl-5-[2-(pyridin-4-ylamino)-ethoxy]-phenyl}-2-butoxy-benzolsulfonamid

erhielt man in 10% Ausbeute analog dem Bsp. 1. wobei man in der Stufe 1i) 2-Butoxy-benzolsulfonylchlorid an Stelle von Benzolsulfonylchlorid einsetzte. Amorph. MS (m/e)= 545.

**Beispiel 4**

N-(Dimethyloxophosphinylmethyl)-N-{3-methyl-5-[2-(pyridin-4-ylamino)-ethoxy]-phenyl}-2 hexyloxy-benzolsulfonamid

erhielt man in 20% Ausbeute analog dem Bsp. 1, wobei man in der Stufe 1i) 2-Hexyloxy-benzolsulfonylchlorid an Stelle von Benzolsulfonylchlorid einsetzte. Amorph. MS (m/e)= 573.

**Beispiel 5**

N-(Dimethyloxophosphinylmethyl)-N-{3-methyl-5-[2-(pyridazin-4-ylamino)-ethoxy]-phenyl)-2 methoxy-benzolsulfonamid

a) 59 g (233 mmol) 2-(3-Hydroxy-5-methyl-phenyl)-isoindol-1,3-dion, 44 mL (700 mmol) Chloracetonitril und 96.7 g (700 mmol) Kaliumcarbonat erhitzte man in 300 mL trockenem Dimethylformamid 4 h auf 80 °C. Man goß auf 2 L Wasser, filtrierte und erhielt 60.5 g (89%) [3-(1,3-Dioxo-1,3-dihydro-isoindol-2-yl)-5-methyl-phenoxy]-acetonitril mit dem Fp. 156 - 157 °C.

b) 30.0 g (103 mmol) [3-(1,3-Dioxo-1,3-dihydro-isoindol-2-yl)-5-methyl-phenoxy]-acetonitril und 6.0 mL (123 mmol) Hydrazinhydrat in 500 mL Ethanol rührte man 4 h bei Raumtemperatur, saugte den Niederschlag ab, digerierte mit Ether und erhielt 16.7 g (quant.) (3-Amino-5-methyl-phenoxy)-acetonitril mit dem Fp. 76 - 77 °C.

c) Zu 8.9 g (55 mmol) (3-Amino-5-methyl-phenoxy)-acetonitril und 7.6 mL (55 mmol) Triethylamin in 70 mL Dichlormethan gab man bei 10 °C portionsweise 11.4 g (55 mmol) 2-Methoxy-benzolsulfonylchlorid, rührte 1 h bei Raumtemperatur. extrahierte mit Wasser. trocknete die organische Phase über Natriumsulfat, filtrierte, entfernte das Lösungsmittel i. Vak, digerierte den Rückstand mit Ether und erhielt 8.5 g (46%) N-(3-Cyanomethoxy-5-methyl-phenyl)-2-methoxy-benzolsulfonamid mit dem Fp. 156 - 157 °C.

d) 5,0 g (15 mmol) N-(3 -Cyanomethoxy-5-methyl-phenyl)-2-methoxy-benzolsulfonamid. 9.5 g (75 mmol) Chlormethyl-dimethyl-phosphanoxid und 10.5 g (75 mmol) Kaliumcarbonat ruhrte man 30 min bei 160 °C. Man extrahierte mit Essigester, filtrierte über Kieselgel (Essigester : Eisessig = 95.5), entfernte etwa 2/3 des Lösungsmittels,

extrahierte mit Natriumhydrogencarbonat, trocknete die Essigesterphase über Natriumsulfat, filtrierte, entfernte das Lösungsmittel i. Vak und erhielt 5.0 g (79%) N-(Dimethyloxophosphinyl-methyl)-N-(3-cyanome-thoxy-5-methyl-phenyl)-2-methoxy-benzolsulfonamid als farblose Kristalle mit dem Fp. 143 - 145 °C.

e) Zu 0.29 g (13.2 mmol) Lithiumborhydrid in 5 mL Tetrahydrofuran tropfte man unter Stickstoff und Eiskühlung 3.3 mL Chlortrimethylsilan, rührte 1 h bei Raumtemperatur, tropfte eine Lösung von 0.93 g (2.2 mmol) N-(Dimethyloxophosphinyl-methyl)-N-(3-cyanomethoxy-5-methyl-phenyl)-2-methoxy-benzolsulfonamid 5 mL Tetrahydrofuran zu, rührte 1 h bei Raumtemperatur, tropfte Wasser zu, entfernte das Lösungsmittel i. Vak., nahm in wenig Dichlormethan auf und filtrierte über Kieselgel (Dichlormethan : Methanol = 9:1), entfernte das Lösungmittel i. Vak, und erhielt 900 mg (91%) N-(Dimethyloxophosphinyl-methyl)-N-[3-(2-amino-ethoxy)-5-methyl-phenyl]-2-methoxy-benzolsulfonamid als Ö1. MS (m/e)= 426.

f) 940 mg N-(Dimethyloxophosphinyl-methyl)-N-[3-(2-amino-ethoxy)-5-methyl-phenyl]-2-methoxy-benzolsulfonamid, 400 mg ( 2.2 mmol) 3,4,5-Trichlorpyridazin und 0.31 mL (2.2 mmol) Triethylamin in 20 mL trockenem Tetrahydrofuran rührte man 2 h bei 80 °C. entfernte das Lösungmittel i. Vak., ruhrte den Rückstand mit Wasser, extrahierte dreimal mit Essigester. trocknete über Natriumsulfat. filtrierte, entfernte das Lösungsmittel i. Vak. und erhielt 1.26 g (quant.) einer Mischung aus N-(Dimethyloxophosphinyl-methyl)-N-{3-[2-(3,5-dichlor-pvridazin-4-ylamino)-ethoxy]-5-methyl-phenyl}-2-methoxy-benzolsulfonamid und N-(Dimethyloxophosphinyl-methyl)-N-{3-[2-(3,4-dichlor-pyridazin-5-ylamino)-ethoxy]-5-methyl-phenyl}-2-methoxy-benzolsulfonamid als Ö1. MS (m/e) = 573.

g) 1.3 g (2.2 mmol) dieser Mischung und 0.9 g (6.6 mmol) Kaliumcarbonat in 80 mL Methanol hydrierte man in Gegenwart von 0.3 g 10% Palladium auf Kohle bei Raumtemperatur und Normaldruck. Man filtrierte uber Kieselgel, entfernte das Lösungsmittel i. Vak. und erhielt 415 mg (38%) der Titelverbindung als Ö1. MS (m/e)= 504.

**Beispiel 6**

N-(Dimethyloxophosphinyl-methyl)-N-{3-methyl-5-[2-(pyridazin-4-ylamino)-ethoxy] -phenyl}-2-propyloxy-benzolsulfonamid

stellte man in 33% Ausbeute analog dem Beispiel 5 her. Ö1. MS (m/e) = 532. Dazu setzte man in Stufe 5c) 2-Propyloxy-benzolsulfonylchlorid an Stelle des 2-Methoxy-benzolsulfonylchlorid ein und erhielt in 57% Ausbeute N-(3-Cyanomethoxy-5-methyl-phenyl)-2-propyloxy-benzolsulfonamid (Fp. 154 - 155 °C), das man analog Beispiel 5d) zu N-(Dimethyloxophosphinyl-methyl)-N-(3 -cyanomethoxy-5-methyl-phenyl)-2-propyloxy-benzolsulfonamid (60% Ausbeute. Fp. 143 - 145 °C), dieses analog Bsp. 5e) zu N-(Dimethyloxophosphinyl-methyl)-N-[3-(2-amino-ethoxy)-5-methyl-phenyl]-2-propyloxy-benzolsulfonamid (86% Ausbeute, Ö1. MS (m/e)= 454), dieses analog Bsp. 5f) zu einer Mischung aus N-(Dimethyloxophosphinyl-methyl)-N-{3-[2-(3,5-dichlor-pyridazin-4-ylamino)-ethoxy]-5-methyl-phenyl}-2-propyloxy-benzolsulfonamid und N-(Dimethyloxophosphinyl-methyl)-N-{3-[2-(3,4-dichlor-pyridazin-5-ylamino)-ethoxy]-5-methyl-phenyl}-2-propyloxy-benzolsulfonamid (quant., Ö1, MS (m/e) = 601) umsetzte, woraus man die Titelverbindung durch katalytische Hydrierung analog Bsp. 5g) erhielt.

**Beispiel 7**

N-(Dimethyloxonhosphinyl-methyl)-N-{3-methyl-5-{2-(pyridazin-4-ylamino)-ethoxy] -phenyl}-2-ethoxy-benzolsulfonamid

stellte man in 54% Ausbeute analog dem Beispiel 5 her. Ö1. MS (m/e)= 518. Dazu setzte man in Stufe 5c) 2-Ethoxy-benzolsulfonylchlorid an Stelle des 2-Methoxy-benzolsulfonylchlorid ein und erhielt in 70% Ausbeute N-(3-Cyanomethoxy-5-methyl-phenyl)-2-ethoxy-benzolsulfonamid (Fp. 142 °C). das man analog Beispiel 5d) zu N-(Dimethyloxophosphinyl-methyl)-N-(3-cyanomethoxy-1-methyl-phenyl)-2-ethoxy-benzolsulfonamid (46% Ausbeute. Fp. 139 - 141 °C). dieses analog Bsp. 5e) zu N-(Dimethyloxophosphinyl-methyl)-N-[3-(2-amino-ethoxy)-5-methyl-phenyl]-2-ethoxybenzolsulfonamid (58% Ausbeute. Ö1. MS (m/e)= 440). dieses analog Bsp. 5f) zu einer Mischung aus N-(Dimethyloxophosphinyl-methyl)-N-{3-[2-(3,5-dichlorpyridazin-4-ylamino)-ethoxy]-5-methyl-phenyl}-2-ethoxy-benzolsulfonamid und N-(Dimethyloxophosphinyl-methyl)-N-{3-[2-(3,4-dichlor-pyridazin-5-ylamino)-ethoxy]-5-methyl-phenyl}-2-ethoxy-benzolsulfonamid (55%. Fp. 114 - 116 °C) umsetzte, woraus man die Titelverbindung durch katalytische Hydrierung analog Bsp. 5g) erhielt.

**Beispiel 8**

N-(Dimethyloxophosphinyl-methyl)-N-{3-methyl-5-[2-(pyridazin-4-ylamino)-ethoxy] -phenyl}-2-(2-propyl-oxy)-benzolsulfonamid

stellte man in 35% Ausbeute analog dem Beispiel 5 her. Ö1. MS (m/e)= 532. Dazu setzte man in Stufe 5c) 2-Isopropyloxy-benzolsulfonylchlorid an Stelle des 2Methoxy-benzolsulfonylchlorid ein und erhielt in 21% Ausbeute N-(3-Cyanomethoxy-5-methyl-phenyl)-2-(2-propyloxy)-benzolsulfonamid (Fp. 100 - 102 °C), das man analog Beispiel 5d) zu N-(Dimethyloxophosphinyl-methyl)-N-(3-cyanomethoxy-5-methyl-phenyl)-2-(2-propyloxy)-benzolsulfonamid (65%, Fp. 137 - 140 °C), dieses analog Bsp. 5e) zu N-(Dimethyloxophosphinyl-methyl)-N-[3-12-amino-ethoxy)-5-methyl-phenyl]-2-(2-propyloxy)-benzolsulfonamid (75%, Ö1. MS (m/e)= 454), dieses analog Bsp. 5f) zu einer Mischung aus N-(Dimethyloxophosphinyl-methyl)-N-{3-[2-(3,5-dichlor-pyridazin-4-ylamino)-ethoxy]-5-methyl-phenyl}-2-(2-propyloxy)-benzolsulfonamid und N-(Dimethyloxophosphinyl-methyl)-N-{3-[2-(3,4-dichlorpyridazin-5-ylamino)-ethoxy]-5-methyl-phenyl}-2-(2-propyloxy)-benzolsulfonamid (quant. Ö1. MS (m/e)= 601) umsetzte. woraus man die Titelverbindung durch katalytische Hydrierung analog Bsp. 5g) erhielt.

**Beispiel 9**

N-(Dimethyloxochosyhinyl-methyl)-N-{3-methyl-5-[2-(pyridazin-4-ylamino)-ethoxy] -phenyl}-2-butoxy-benzolsulfonamid

stellte man in 41% Ausbeute analog dem Beispiel 5 her. Ö1. MS (m/e) = Dazu setzte man in Stufe 5c) 2-Butoxy-benzolsulfonylchlorid an Stelle des 2-Methoxy-benzolsulfonylchlorid ein und erhielt in 96% Ausbeute N-(3-Cyanomethoxy-5-methylphenyl)-2-ethoxy-benzolsulfonamid (Fp. 121 - 123 °C), das man analog Beispiel 5d) zu N-(Dimethyloxophosphinyl-methyl)-N-(3-cyanomethoxy-5-methyl-phenyl)-2-butoxy-benzolsulfonamid (43% Ausbeute, Fp. 151 - 152 °C), dieses analog Bsp. 5e) zu N-(Dimethyloxophosphinyl-methyl)-N-[3-(2-amino-ethoxy)-5-methyl-phenyl]-2-butoxy-benzolsulfonamid (67% Ausbeute, Ö1, MS (m/e)= 468), dieses analog Bsp. 5f) zu einer Mischung aus N-(Dimethyloxophosphinyl-methyl)-N- {3-[2-(3,5-dichlorpyridazin-4-ylamino)-ethoxy]-5-methyl-phenyl}-2-butoxy-benzolsulfonamid und N-(Dimethyloxophosphinyl-methyl)-N-{3-[2-(3,4-dichlor-pyridazin-5-ylamino)-ethoxy]-5-methyl-phenyl}-2-butoxy-benzolsulfonamid (quant.. Ö1. MS (m/e) = 615) umsetzte, woraus man die Titelverbindung durch katalytische Hydrierung analog Bsp. 5g) erhielt.

**Beispiel 10**

Thrombinzeit

Ein in der klinischen Gerinnungsdiagnostik gebräuchlicher Test ist die Thrombinzeit. Dieser Parameter erfaßt die Thrombinwirkung auf Fibrinogen und die Gerinnselbildung, inhibitoren von Thrombin bewirken eine Verlängerung der Thrombinzeit.

Zur Plasmagewinnung wurden 9 Teile frisches Blut gesunder Spender mit einem Teil Natriumcitratlösung (0,11 Mol/L) gemischt und bei ca. 3000 U/min 10 min bei Raumtemperatur zentrifugiert. Das Plasma wurde abpipettiert und kann bei Raumtemperatur ca. 8 h aufbewahrt werden.

200 μL Citratplasma wurden in einem Kugelkoagulometer(KC10 der Firma Amelung) 2 min bei 37 °C inkubiert. Zu 190 μL vortemperiertem Thrombin-Reagenz (Boehringer Mannheim GmbH; enthält ca. 3 U/mL Pferdethrombin und 0.0125 M $Ca^{++}$) gab man 10 μL Dimethylsulfoxid (DMSO) oder eine Lösung der Wirksubstanz in DMSO. Mit Zugabe dieser 200 μL Lösung zum Plasma wurde eine Stoppuhr gestartet und der Zeitpunkt bis zum Eintritt der Gerinnung bestimmt. Die Thrombinzeit betrug bei den Kontrollmessungen ca. 24 sec. und wurde durch die Wirksubstanzen deutlich verlängert.

In der folgenden Tabelle sind die gemessenen Thrombinzeiten in Sekunden als Differenz zur Kontrolle angegeben. Die Konzentrationen der Wirksubstanzen im Humanplasma betrugen 5 μM (TT5), und 0.5 μM (TT0.5).

Thrombin-Inhibierung

Die kinetischen Messungen wurden in 0.1 M Phosphatpuffer, der 0.2 M Kochsalz und 0.5 % Polyethylenglycol 6000 enthielt, bei einem pH = 7.5 und 25 °C mit dem Substrat H-(D)-Phe-Pro-Arg-pNA; Kabi) und humanem a-Thrombin (Sigma, spezifische Aktivität = 2150 NIH-units/mg) in Polystyrol-Halbmikroküvetten in einem Gesamtvolumen von 1 mL durchgeführt.

In einem Vorversuch wurde mit jeder Wirksubstanz bestimmt, ob sie Thrombin schnell oder langsam inhibiert.

Dazu wurde die Reaktion einmal durch Zugabe von 0.03 NIH-units Thrombin zu einer 100 µM-Lösung des Substrats und des Wirkstoffs gestartet. In einem zweiten Versuch wurde Substrat zu einer 5 min inkubierten Lösung des Thrombins und des Wirkstoffs gegeben. Die Zunahme der Konzentration von p-Nitroanilin mit der Zeit wurde spektroskopisch (UV-VIS-Spektrophotometer Lambda-2 der Firma Perkin-Elmer) bei 405 nm 12 min verfolgt.

Da die bei beiden Versuchen erhaltenen Messkurven linear und parallel waren, handelt es sich bei den Wirkstoffen der folgenden Tabelle um schnelle Thrombin-Inhibitoren.

Die Inhibitionskonstanten $K_i$ wurden dann wie folgt bestimmt. Das Substrat wurde in den Konzentrationen 100 µM, 50 µM, 30 µM, 20 µM eingesetzt und bei jeder Substratkonzentration eine Messung ohne Inhibitor und drei Messungen in Gegenwart unterschiedlicher Konzentrationen der in der folgenden Tabelle aufgeführten Inhibitoren durchgeführt. Die Reaktionen wurden durch Zugabe von Thrombin gestartet. Die Zunahme der Extinktion bei 405 nm durch das entstehende p-Nitroanilin über einen Zeitraum von 12 min verfolgt. Im Abstand von 20 sec wurden Messpunkte (Zeit vs. Extinktion) auf einen PC übertragen. Aus den Daten wurde die Geschwindigkeiten $V_0$ (Extinktionsänderung pro sec; Messungen ohne Inhibitor) und $V_i$ (Messungen mit Inhibitor) durch lineare Regression bestimmt. Benutzt wurde nur der Teil jeder Messung, bei dem sich die Substratkonzentration um weniger als 15% vermindert hatte. Aus einer Meßreihe (konstante Inhibitorkonzentration, variable Substratkonzentrationen) bestimmte man $K_m$' m und $V_{max}$ durch nichtlinearen Fit auf die Gleichung

$$V = \frac{V_{max} * [S]}{[S] + K_m'}$$

Aus den gesamten Meßreihen berechnete man schließlich $K_i$ durch nichtlinearen Fit auf die Gleichung

$$V = \frac{V_{max} * [S]}{K_m*(1+[S]/K_i)+[S]}$$

Die Michaeliskonstante $K_m$ betrug in allen Messungen 3.8 ± 2 µM.

Die inhibitionskonstanten $K_i$ der Wirksubstanzen sind in der folgenden Tabelle in der Einheit µM angegeben.

Inhibierung von Trypsin und Plasmin

10 mg Bovines pancreatisches Trypsin (Sigma) wurden in 100 mL 1 mM Salzsäure gelöst und im Kühlschrank aufbewahrt. 20 µL davon wurden mit 980 µL 1 mM Salzsäure versetzt. 25 µL davon wurde für jede Messung verwendet. Die Messung wurde wie für Thrombin beschrieben durchgeführt. $K_m$ = 45 µM.

Die Messungen mit humanem Plasmin (Sigma, 10 Units) wurden mit dem Substrat S-2251 (H-(D)-Val-Leu-Lys-pNA, Kabi) wie für Thrombin beschrieben durchgeführt. Pro Messung wurden 0.01 Units Plasmin verwendet. $K_m$ = 250 µM.

| Verbindungen aus Beispiel | TT5 | TT0.5 | Ki [µM] Thrombin |
|---|---|---|---|
| 1 | 175 | 33 | 0.100 |
| 2 | 300 | 115 | 0.008 |
| 3. | 300 | 50 | 0.004 |
| 4 | 113 | 11 | 0.012 |
| 5 | 162 | 32 | 0.042 |
| 6 | 122 | 18 | 0.028 |
| 7 | 109 | 14 | 0.060 |
| 8 | 180 | 47 | 0.035 |

Eine Trypsin- und Plasmininhibierung wurde für die erfindungsgemäßen Verbindungen nicht festgestellt.

**Patentansprüche**

**1.** Phosphanoxide der allgemeinen Formel I

(I)

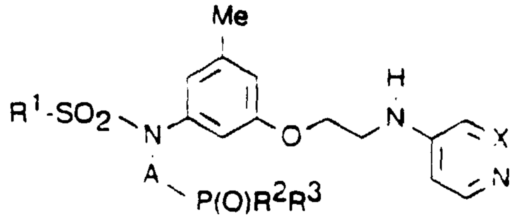

in der

| | |
|---|---|
| $R^1$ | eine Aryl- oder Heteroarylgruppe bedeutet, wobei die Aryl- oder Heteroarylreste ein- oder mehrfach durch Nitro, Halogen Nitril, Hydroxy, Carboxy, Alkoxycarbonyl, Phenylalkoxycarbonyl, Phenyl, Alkyl, Trifluormethyl, Alkoxy, Alkenyloxy, Alkinyloxy, Aralkyloxy, Alkylthio, Alkylsulfinyl, Alkylsulfonyl, Amino, Alkylamino, Dialkylamino, Aralkylamino, Diaralkyl-amino, Alkylsulfonylamino, Alkylcarbonylamino, Formylamino, Carbamoyl, Thiocarbamoyl, Alkylaminocarbonyl, Dialkylaminocarbonyl oder Alkoxycarbonylalkyloxy substituiert sein können. |
| $R^2$ und $R^3$ | gleich oder verschieden sind und geradkettige oder verzweigte Alkylgruppen bedeuten, |
| A | einen geradkettigen oder verzweigten Alkylenrest bedeutet, |
| X | die -CH-Gruppe oder ein Stickstoffatom bedeutet. |

sowie Hydrate, Solvate und physiologisch verträgliche Salze davon. Gegenstand der Erfindung sind auch die optisch aktiven Formen, die Racemate und die Diastereomerengemische dieser Verbindungen.

2. Phosphanoxide der allgemeinen Formel I gemäß Anspruch 1, in der $R^1$ eine unsubstituierte oder durch eine $C_1$-$C_6$-Alkoxyuruppe substituierte Phenylgruppe bedeutet.

3. Phosphanoxide der allgemeinen Formel I gemäß den Anspruchen 1 und 2. in der $R^1$ und $R^2$ gleich sind und $C_1$-$C_6$-Alkylgruppen bedeuten.

4. Phosphanoxide der allgemeinen Formel I gemaß den Anspruchen 1 bis 3, in der A eine $C_1$-$C_6$-Alkylengruppe bedeutet.

5. Phosphanoxide der allgemeinen Formel I gemaß den Anspruchen 1 bis 4, in der X eine -CH-Gruppe oder ein Stickstoffatom bedeutet.

6. Verfahren zur Herstellung von Phosphanoxiden der allgemeinen Formel I gemäß den Anspruchen 1 bis 5, worin man eine Verbindung der allgemeinen Formel III

III

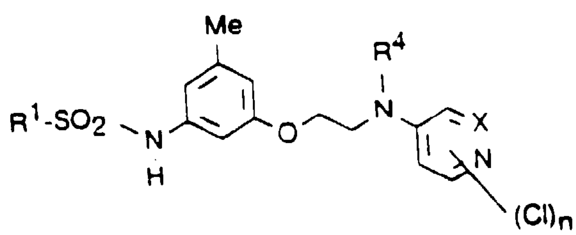

in der $R^1$ und X die oben angegebene Bedeutungen haben. $R^4$ ein Wasserstoffatom oder die Benzylgruppe ist und n gleich 2 oder 4 ist, mit den Phosphanoxiden Hal-A-P(O)$R^2R^3$, in denen A, $R^2$ und $R^3$ die oben angegebenen Bedeutungen haben und Hal ein Halogenatom bedeutet, umsetzt und anschließend katalytisch hydriert und dann gewünschtenfalls die Verbindungen der allgemeinen Formel I in physiologisch verträgliche Salze, Hydrate, Solvate, oder optische Isomere umwandelt.

7. Pharmazeutische Zubereitungen, die mindestens eine der Verbindungen der allgemeinen Formel I gemäß den Ansprüchen 1 bis 5 enthalten und zusätzlich einen pharmazeutischen Träger und Hilfsstoffe.

8. Verwendung von Verbindungen der allgemeinen Formel I zur Herstellung von Arzneimitteln zur Behandlung thromboembolischer Erkrankungen.

## Claims

1. Phosphane oxides of the general formula I

(I)

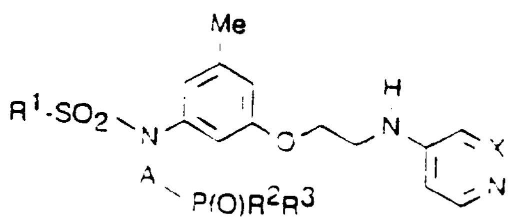

in which

| | |
|---|---|
| $R^1$ | denotes an aryl or heteroaryl group in which the aryl or heteroaryl residues can be substituted once or several times by nitro, halogen, nitrile, hydroxy, carboxy, alkoxycarbonyl, phenylalkoxycarbonyl, phenyl, alkyl, trifluoromethyl, alkoxy, alkenyloxy, alkinyloxy, aralkyloxy, alkylthio, alkylsulfinyl, alkylsulfonyl, amino, alkylamino, dialkylamino, aralkylamino, di-aralkylamino, alkylsulfonylamino, alkylcarbonylamino, formylamino, carbamoyl, thiocarbamoyl, alkylaminocarbonyl, dialkylaminocarbonyl or alkoxycarbonylalkyloxy, |
| $R^2$ and $R^3$ | are the same or different and denote straight-chain or branched alkyl groups, |
| A | denotes a straight-chain or branched alkylene residue, |
| X | denotes a -CH group or a nitrogen atom, |

as well as hydrates, solvates and physiologically tolerated salts thereof. The invention also concerns the optically active forms, racemates and mixtures of diastereomers of these compounds.

2. Phosphane oxides of the general formula I as claimed in claim 1, in which $R^1$ denotes an unsubstituted phenyl group or a phenyl group substituted by a $C_1$-$C_6$ alkoxy group.

3. Phosphane oxides of the general formula I as claimed in claims 1 and 2, in which $R^1$ and $R^2$ are the same and denote $C_1$-$C_6$ alkyl groups.

4. Phosphane oxides of the general formula I as claimed in claims 1 to 3, in which A denotes a $C_1$-$C_6$ alkylene group.

5. Phosphane oxides of the general formula I as claimed in claims 1 to 4, in which X denotes a -CH group or a nitrogen atom.

6. Process for the production of phosphane oxides of the general formula I as claimed in claims 1 to 5, wherein a compound of the general formula III

III

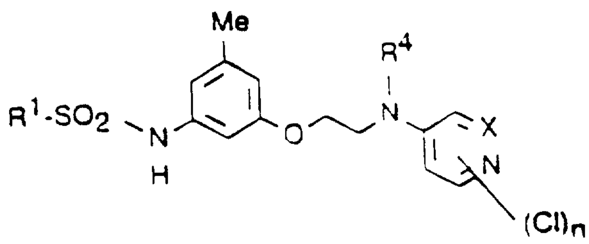

in which $R^1$ and X have the above-mentioned meanings, $R^4$ is a hydrogen atom or a benzyl group and n equals 2 or 4 is reacted with the phosphane oxides Hal-A-P(O)$R^2R^3$ in which A, $R^2$ and $R^3$ have the above-mentioned meanings and Hal denotes a halogen atom and it is subsequently catalytically hydrogenated
and then if desired the compounds of the general formula I are converted into physiologically tolerated salts, hydrates, solvates or optical isomers.

**7.** Pharmaceutical preparations which contain at least one of the compounds of the general formula I as claimed in claims 1 to 5 in addition to a pharmaceutical carrier and auxiliary substances.

**8.** Use of compounds of the general formula I to produce pharmaceutical preparations for the treatment of thromoboembolic diseases.

**Revendications**

**1.** Phosphanoxydes de formule générale I

(I)

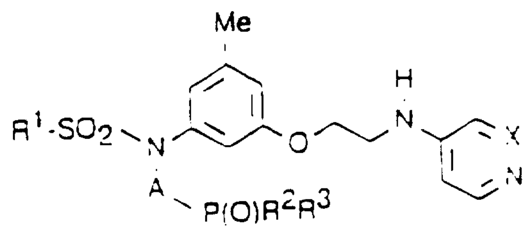

dans laquelle

$R^1$ représente un groupe aryle ou hétéroaryle, où les restes aryle ou hétéroaryle peuvent être substitués une ou plusieurs fois par un groupe nitro, halogéno, nitrile, hydroxy, carboxy, alcoxycarbonyle, phénylalcoxycarbonyle, phényle, alkyle, trifluorométhyle, alcoxy, alcényloxy, alcinyloxy, aralkyloxy, alkylthio, alkylsulfinyle, alkylsulfonyle, amino, alkylamino, dialkylamino, aralkylamino, diaralkylamino, alkylsulfonylamino, alkylcarbonylamino, formylamino, carbamoyle, thiocarbamoyle, alkylammocarbonyle, dialkylaminocarbonyle ou alcoxycarbonylalkyloxy,

$R^2$ et $R^3$ sont identiques ou différents et représentent des groupes alkyle à chaîne linéaire ou ramifiée,

A représente un reste alkyle à chaîne linéaire ou ramifiée,

X représente le groupe -CH ou un atome d'azote,

ainsi que les hydrates, les formes solvatées et les sels physiologiquement acceptables de ces composés. L'invention a également pour objet les formes optiquement actives, les racémates et les mélanges de diastéréomères de ces composés.

**2.** Phosphanoxydes de formule générale I selon la revendication 1, où $R^1$ représente un groupe phényle non substitué ou substitué par un groupe alcoxy en $C_1$-$C_6$.

3. Phosphanoxydes de formule générale I selon les revendications 1 et 2, où $R^1$ et $R^2$ sont identiques et représentent un groupe alkyle en $C_1$-$C_6$.

4. Phosphanoxydes de formule générale I selon les revendications 1 à 3, où A représente un groupe alkylène en $C_1$-$C_6$.

5. Phosphanoxydes de formule générale I selon les revendications 1 à 4, où X représente un groupe -CH ou un atome d'azote.

6. Procédé de préparation de phosphanoxydes de formule générale I selon les revendications 1 à 5, dans lequel on fait réagir un composé de formule générale III

III

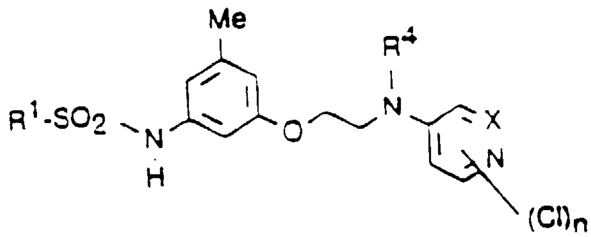

dans laquelle $R^1$ et X ont les significations indiquées ci-dessus, $R^4$ représente un atome d'hydrogène ou un groupe benzyle et n vaut 2 ou 4, avec les phosphanoxydes Hal-A-P(O)$R^2R^3$, où A, $R^2$ et $R^3$ ont les significations indiquées ci-dessus et Hal représente un atome d'halogène, et ensuite, on soumet le produit obtenu à une hydrogénation catalytique,
et ensuite on transforme, le cas échéant, les composés de formule générale I en leurs sels, hydrates, formes solvatées ou isomères optiques physiologiquement acceptables.

7. Préparations pharmaceutiques, qui contiennent au moins un des composés de formule générale I selon les revendications 1 à 5, et en plus, un véhicule et des adjuvants pharmaceutiques.

8. Utilisation de composés de formule générale I pour la préparation de médicaments destinés au traitement de maladies thromboemboliques.